# DEMANDE DE BREVET EUROPEEN

(11) **EP 4 494 647 A1**
(43) Date de publication de la demande: **22.01.2025**
(21) Numéro de dépôt: 24189628.1
(22) Date de dépôt: 19.07.2024
(51) Int. Cl.: A61K 36/28, A61P 17/00, A61K 8/9789, A61Q 19/00, A61K 9/00

(54) **EXTRAIT D AKÈNES DE SILYBUM MARIANUM (L.) GAERTN. POUR LE TRAITEMENT OU LA PRÉVENTION DE ROUGEURS CUTANÉES**

(30) Priorité: 21.07.2023 FR 2307825
(71) Demandeur: Pierre Fabre Dermo-Cosmétique, 81500 Lavaur (FR)
(72) Inventeur: SAURAT, Jean-Hilaire, 1206 GENEVE (CH)
(74) Mandataire: Regimbeau

(57) **Abrégé**

La présente invention a pour objet un extrait d'akènes de *Silybum marianum* (L.) Gaertn. comprenant moins de 0,2% en poids de silymarine par rapport au poids de l'extrait sec, pour son utilisation dans le traitement ou la prévention des rougeurs cutanées.

La présente invention a également pour objet une composition dermatologique ou cosmétique comprenant un tel extrait en mélange avec au moins un excipient dermatologiquement ou cosmétiquement acceptable pour son utilisation dans le traitement des rougeurs cutanées.

## Description

### DOMAINE DE L'INVENTION

La présente invention se rapporte à un extrait d'akènes de *Silybum marianum* (L.) Gaertn. comprenant moins de 0,2%, de préférence moins de 0,1%, en poids de silymarine par rapport au poids de l'extrait sec pour son utilisation dans le traitement ou la prévention de rougeurs cutanées, c'est-à-dire de la peau, incluant le cuir chevelu, notamment les rougeurs liées au stress de l'environnement ou encore les rougeurs associées à un désordre cutané tel que la rosacée.

### ETAT DE LA TECHNIQUE

La peau est l'organe du corps humain le plus étendu, ayant une surface de près de 2m². Elle joue plusieurs rôles fondamentaux dont celui de protection vis-à-vis de l'extérieur, de régulation thermique, de synthèse hormonale et elle possède aussi une fonction immunitaire. La peau est constituée de trois couches superposées que sont l'épiderme, en renouvèlement permanent, le derme et l'hypoderme qui assure la charpente fibreuse. La peau, par sa couleur, sa texture et sa qualité, possède en outre une incontestable fonction psychosociale.

Les peaux sensibles ont en commun de réagir rapidement et excessivement à un changement de température, au froid notamment, au vent, à des produits d'hygiène ou de toilette irritants, à des soins inadaptés. Les rougeurs du visage sont l'apanage des peaux sensibles, ces peaux qui se singularisent par une réactivité exacerbée de la peau et des vaisseaux cutanés. Les rougeurs sont plus ou moins intermittentes, mais toujours désagréables et gênantes.

La rougeur intermittente, encore dénommée « flush », est une réaction de vasodilatation aiguë qui peut être due à une situation banale ou un peu stressante, telle qu'un examen pour un étudiant, un entretien d'embauche, une conversation affectivement émouvante ou conflictuelle mais encore à un changement de température qui sollicite la circulation du sang au niveau du visage. Une alimentation trop chaude, des boissons alcoolisées ou certains aliments (épices, moutarde ...) peuvent aussi causer des rougeurs.

Différents facteurs peuvent favoriser et aggraver ces rougeurs : les facteurs externes, comme le froid ou le soleil, qui entraînent une accélération de la microcirculation cutanée ; les facteurs internes, comme les émotions, la consommation de café ou d'épices ; l'hérédité, les rougeurs peuvent apparaitre chez des personnes ayant une peau vaso-réactive, une caractéristique cutanée qui peut être familiale ; ou encore le vieillissement cutané car, dès 25 ans, la peau est plus sensible aux rougeurs.

A la longue, les rougeurs peuvent devenir permanentes, notamment au niveau des joues. C'est ce qu'on appelle l'érythrose qui constitue une rougeur diffuse mais permanente localisée le plus souvent sur les joues sous forme de plaques rouges.

La couperose représente une aggravation de l'érythrose avec apparition de petits vaisseaux dilatés.

La rosacée est une dermatose commune chronique et progressive liée notamment à une relaxation vasculaire. Il s'agit d'une affection qui touche les petits vaisseaux du visage. Elle touche fréquemment les personnes à peau claire et peut entraîner des conséquences psychoaffectives importantes. Le nom de cette pathologie fait référence à la couleur caractéristique que prend le visage lors de la maladie. L'aspect général du visage faisant évoquer à tort un excès chronique de consommation d'alcool, la rosacée est une affection socialement difficile à vivre, en particulier par les femmes. La rosacée est une pathologie plutôt fréquente, puisqu'en France, 2 à 3% de la population adulte est concernée. Les femmes sont particulièrement touchées, puisque l'on compte deux femmes pour un homme souffrant de rosacée. Elle affecte principalement la partie centrale du visage et se caractérise par un rougissement du visage ou des bouffées de chaleur, un érythème facial, des papules, des pustules, une télangiectasie et parfois des lésions oculaires appelées rosacée oculaire. Dans des cas graves, particulièrement chez l'homme, le tissu mou du nez peut enfler et produire un gonflement bulbeux appelé rhinophyma. La rosacée évolue, mais de façon non obligatoire, sur plusieurs années par poussées aggravées par différents stimuli comme les variations de température, l'alcool, les épices, l'exposition solaire ou les émotions.

Ainsi, de façon générale, la rosacée évolue le plus souvent en 4 stades :
- Stade 1 : il s'agit de relaxations vasculaires ayant une composante neurogène (plutôt vers 20 ans). Les patients ont des poussées soudaines de rougeur paroxystique du visage et du cou, avec des sensations de chaleur, mais sans signes systémiques. Après les crises, la peau du visage redevient normale. Ces « flushs » sont déclenchés par les changements de température, l'absorption de boissons chaudes ou d'alcool.
- Stade 2 : on parle de stade érythémato-télangiectasique ou encore stade vasculaire (plutôt vers 30 ans). Les zones malaires sont diffusément rouges. On y observe des capillaires dilatés constituant la classique couperose. A la différence du stade 1, la rougeur est permanente. En plus des joues, le menton et la partie médiane du front peuvent être affectés.
- Stade 3 : c'est le stade des papulo-pustules ou encore le stade inflammatoire (plutôt vers 40 ans). Sur fond d'érythème se développent des papules et des pustules de quelques millimètres de diamètre, sans comédons associés. Cette dermatose peut être très étendue, parfois à la partie glabre du cuir chevelu chez l'homme, mais respecte le contour de la bouche et des yeux. Les patients se plaignent d'une peau sensible, avec une intolérance subjective à la plupart des produits cosmétiques.
- Stade 4 : il est appelé le rhinophyma (plutôt à partir de 50 ans). Cette phase tardive touche principalement les hommes, contrairement aux autres stades. Le nez est augmenté de volume, diffusément rouge et les orifices folliculaires sont dilatés. La peau s'épaissit progressivement.

Malgré sa fréquence, ses causes restent encore mal déterminées, et peuvent impliquer plusieurs facteurs. L'alimentation et les facteurs climatiques jouent un rôle dans la maladie et ont un impact sur ces symptômes. Il existe parfois, chez des patients atteints de rosacée, un dysfonctionnement des veines du visage, qui se traduit par une stagnation du sang dans les vaisseaux de la face, entraînant en cascade la dilatation des vaisseaux, l'oedème et l'altération de l'endothélium. Les corticoïdes peuvent également être incriminés, notamment dans 2 situations. La première c'est la rosacée qui est aggravée par l'application de corticoïdes. La seconde c'est l'apparition de la maladie suite à l'utilisation au long cours de ce type de médicaments, on parle même de rosacée stéroïdienne.

Récemment, il a été montré que la voie de signalisation du récepteur tyrosine kinase Tieangiopoiétine régulait la perméabilité vasculaire et participait au remodelage vasculaire durant des situations pathologiques (Saharinen et al. 2017). L'angiopoiétine représente une famille importante de facteurs de croissance, ses activités étant médiées par 2 récepteurs de type tyrosine kinase : Tie1 et Tie2.

L'angiopoiétine 1 est un puissant facteur de croissance angiogénique, son effet passant par Tie2. L'angiopoiétine 1 produite par les péricytes se lie à son récepteur Tie2 sur la membrane des cellules endothéliales. Cette activation permanente permet la stabilisation du capillaire : on parle de quiescence vasculaire.

Récemment, il a été montré que l'angiopoiétine 2 avait également un rôle dans la physiopathologie vasculaire (Akwii et al. 2019). Encore plus récemment, des chercheurs ont montré que l'angiogénèse est un processus essentiel dans la rosacée (Lee et al. 2021).

Les traitements de la rosacée par voie orale à base de dérivés de tétracycline existant actuellement sur le marché sont problématiques pour plusieurs raisons mais en particulier pour leurs effets secondaires non négligeables. L'administration orale de tétracyclines, comme par exemple la doxycycline, peut induire une photosensibilité, voire même une phototoxicité ou encore des désordres gastro-intestinaux.

Des traitements topiques de la rosacée existent également sur le marché à base de composé nitro-imidazole tel que le métronidazole. Cependant, il est connu que l'utilisation de tels composés peut induire des troubles physiologiques, notamment gastro-intestinaux et rénaux (Edwards 1980).

Cependant, ces traitements ne visent pas directement les rougeurs, le côté vasculaire de la rosacée n'est à ce jour pas couvert thérapeutiquement.

Il existe donc un besoin de disposer de nouveaux actifs pour lutter contre les rougeurs cutanées, notamment chez des patients atteints de rosacée, et en particulier la rosacée érythémato-télangiectasique, qu'ils soient utilisables pendant de longues périodes avec des effets secondaires aussi limités que possible.

### RESUME DE L'INVENTION

La présente invention a pour objet de répondre à ces besoins. En effet, les inventeurs ont mis en évidence, de façon tout à fait inattendue, qu'un extrait d'akènes de *Silybum marianum* (L.) Gaertn. comprenant moins de 0,2%, de préférence moins de 0,1%, en poids de silymarine par rapport au poids de l'extrait sec avait la capacité de réduire significativement les rougeurs de patients atteints de rosacée érythémato-télangiectasique, en agissant sur la voie de signalisation du récepteur tyrosine kinase Tie2/angiopoiétine.

La présente invention a donc pour objet un extrait d'akènes de *Silybum marianum* (L.) Gaertn. comprenant moins de 0,2%, de préférence moins de 0,1%, en poids de silymarine par rapport au poids de l'extrait sec, pour son utilisation dans le traitement ou la prévention de rougeurs cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également l'utilisation d'un extrait d'akènes de *Silybum marianum* (L.) Gaertn. comprenant moins de 0,2%, de préférence moins de 0,1%, en poids de silymarine par rapport au poids de l'extrait sec, dans le traitement ou la prévention des rougeur cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également l'utilisation d'un extrait d'akènes de *Silybum marianum* (L.) Gaertn. comprenant moins de 0,2%, de préférence moins de 0,1%, en poids de silymarine par rapport au poids de l'extrait sec, pour la préparation d'un médicament destiné au traitement ou à la prévention de rougeurs cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également une méthode de traitement ou de prévention de rougeurs cutanées, comprenant l'administration à une personne en ayant besoin, en particulier d'une personne atteinte de rosacée, notamment de rosacée érythémato-télangiectasique, et/ou de dermatite atopique, en particulier chez des patients atteints de rosacée, notamment de rosacée érythémato-télangiectasique, d'une quantité efficace d'un extrait d'akènes de *Silybum marianum* (L.) Gaertn. comprenant moins de 0,2%, de préférence moins de 0,1%, en poids de silymarine par rapport au poids de l'extrait sec.

La présente invention a également pour objet une composition cosmétique ou dermatologique comprenant un extrait d'akènes de *Silybum marianum* (L.) Gaertn. comprenant moins de 0,2%, de préférence moins de 0,1%, en poids de silymarine par rapport au poids de l'extrait sec, avec au moins un excipient cosmétiquement ou dermatologiquement acceptable, pour son utilisation dans le traitement ou la prévention de rougeurs cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également l'utilisation d'une composition cosmétique ou dermatologique comprenant un extrait d'akènes de *Silybum marianum* (L.) Gaertn. comprenant moins de 0,2%, de préférence moins de 0,1%, en poids de silymarine par rapport au poids de l'extrait sec, avec au moins un excipient cosmétiquement ou dermatologiquement acceptable, dans le traitement ou la prévention de rougeurs cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également l'utilisation d'une composition cosmétique ou dermatologique comprenant un extrait d'akènes de *Silybum marianum* (L.) Gaertn. comprenant moins de 0,2%, de préférence moins de 0,1%, en poids de silymarine par rapport au poids de l'extrait sec, avec au moins un excipient cosmétiquement ou dermatologiquement acceptable, pour la préparation d'un médicament destiné au traitement ou à la prévention de rougeurs cutanées, en particulier chez des patients atteints de rosacée, notamment de rosacée érythémato-télangiectasique, et/ou de dermatite atopique, en particulier chez des patients atteints de rosacée, notamment de rosacée érythémato-télangiectasique.

La présente invention concerne également une méthode de traitement ou de prévention de rougeurs cutanées comprenant l'administration à une personne en ayant besoin, en particulier d'une personne atteinte de rosacée, notamment de rosacée érythémato-télangiectasique, et/ou de dermatite atopique, en particulier chez des patients atteints de rosacée, notamment de rosacée érythémato-télangiectasique, d'une quantité efficace d'une composition cosmétique ou dermatologique comprenant un extrait d'akènes de *Silybum marianum* (L.) Gaertn. comprenant moins de 0,2%, de préférence moins de 0,1%, en poids de silymarine par rapport au poids de l'extrait sec, avec au moins un excipient cosmétiquement ou dermatologiquement acceptable.

La présente invention concerne également une méthode pour réguler la voie de signalisation du récepteur tyrosine kinase Tie2/angiopoiétine comprenant l'administration à une personne en ayant besoin d'une quantité efficace d'un extrait d'akènes de *Silybum marianum* (L.) Gaertn. comprenant moins de 0,2%, de préférence moins de 0,1%, en poids de silymarine par rapport au poids de l'extrait sec ou d'une composition cosmétique ou dermatologique comprenant un extrait d'akènes de *Silybum marianum* (L.) Gaertn. comprenant moins de 0,2%, de préférence moins de 0,1%, en poids de silymarine par rapport au poids de l'extrait sec, avec au moins un excipient cosmétiquement ou dermatologiquement acceptable.

L'extrait d'akènes de *Silybum ma*rianum (L.) Gaertn. utilisé dans le cadre de la présente invention est de préférence obtenu ou susceptible d'être obtenu par un procédé comprenant une étape d'extraction d'une huile issue d'akènes de *Silybum marianum* (L.) Gaertn. avec un solvant d'extraction comprenant une solution aqueuse hydrotropique, de l'eau subcritique ou un solvant organique non miscible à l'huile issue d'akènes de *Silybum marianum* (L.) Gaertn. éventuellement en mélange avec de l'eau.

### DESCRIPTION DETAILLEE

### Définitions

Dans la présente invention, la plante *Silybum marianum* (L.) Gaertn. pourra être désignée de manière abrégée par le terme *Silybum marianum.*

Par « silymarine », on entend, au sens de la présente invention un extrait purifié d'akènes de *Silybum marianum* (L.) Gaertn. comprenant majoritairement (au moins 95% en poids) un mélange des quatre flavonolignanes suivants : silybine, isosilybine, silychristine et silydianine (Kuki et al. 2012). Une teneur en silymarine inférieure à 0,2% en poids signifie donc que la quantité totale des constituants de la silymarine est inférieure à 0,2% en poids. Une telle teneur peut être déterminée notamment par HPLC (chromatographie en phase liquide à haute performance) ou UPLC (chromatographie en phase liquide à ultra haute performance) en calculant l'aire totale des pics correspondant à tous les constituants de la silymarine, notamment en utilisant un échantillon de silymarine de référence, qui peut être obtenu par exemple auprès de Sigma Aldrich, pour déterminer la position des pics.

Par « silybine », encore appelée dans l'art silibinine, on entend, au sens de la présente invention, les quatre diastéréoisomères silybine A, silybine B, 2,3-cis-silybine A et 2,3-cis-silybine B.

Par « isosilybine », on entend, au sens de la présente invention, les deux diastéréoisomères isosilybine A et isosilybine B.

Par « silychristine », on entend, au sens de la présente invention, les deux diastéréoisomères silychristine A et silychristine B.

Par « stérol », on entend, au sens de la présente invention, une molécule possédant un noyau de stérane dont le carbone 3 est porteur d'un groupe hydroxyle OH, le stérane ayant la structure suivante :

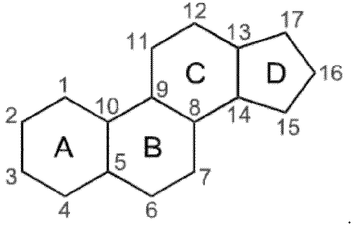

Par « acide gras », on entend, au sens de la présente invention, un acide carboxylique de formule R¹CO₂H dont la chaîne R¹ est une chaîne hydrocarbonée linéaire ou ramifiée, saturée ou comprenant des doubles liaisons C=C, l'acide carboxylique comprenant de 16 à 22 atomes de carbone (incluant l'atome de carbone de la fonction acide carboxylique).

Par acide gras (incluant l'acide linoléique) « libre », on entend, au sens de la présente invention, un acide gras non lié à d'autres molécules (par ex. à du glycérol ou des dérivés de celui-ci pour donner des glycérides ou à un alcool pour donner un ester gras).

Par « tocophérol », on entend, au sens de la présente invention, l'α-tocophérol, le β-tocophérol, le γ-tocophérol et le δ-tocophérol.

Par « environ », on entend, au sens de la présente invention, que la valeur concernée peut être inférieure ou supérieure de 10%, notamment de 5%, en particulier de 1%, à la valeur indiquée. Par « rougeur cutanée », on entend, au sens de la présente invention une coloration rosée à rouge, voire rouge foncé, de toute ou partie de la peau du corps, incluant le cuir chevelu, ou des muqueuses, de préférence de la peau du visage. Cette manifestation, encore appelée érythème, est le plus souvent indésirable, en particulier lorsqu'elle est associée à d'autres symptômes d'un désordre cutané tel que la rosacée.

Par « traiter » ou « traitement » des rougeurs cutanées, on entend notamment la disparition, l'amélioration, la réduction et/ou l'atténuation des rougeurs. La réduction et/ou l'atténuation des rougeurs inclut notamment la réduction de l'intensité ou de la sévérité des rougeurs et/ou de la zone touchée par les rougeurs.

Par « prévenir » ou « prévention » des rougeurs cutanées, on entend notamment le fait de diminuer le risque d'apparition des rougeurs, de retarder l'apparition des rougeurs, de ralentir la progression des rougeurs, de réduire la fréquence des rougeurs.

Par « flush », on entend, au sens de la présente invention, une rougeur cutanée intermittente, c'est-à-dire non permanente. Elle peut être due à une situation banale ou un peu stressante, telle qu'un examen pour un étudiant, un entretien d'embauche, une conversation affectivement émouvante ou conflictuelle mais encore à un changement de température qui sollicite la circulation du sang au niveau du visage. Une alimentation trop chaude, des boissons alcoolisées ou certains aliments (épices, moutarde ...) peuvent aussi être à l'origine de flushs. Elle peut être également de nature pathologique et résulter par exemple d'un désordre vasculaire comme dans le cadre de la rosacée et en particulier de la rosacée érythémato-télangiectasique.

Par « dermatologiquement ou cosmétiquement acceptable », en entend, au sens de la présente invention, ce qui est utile dans la préparation d'une composition dermatologique ou cosmétique, qui est généralement sûr, non toxique et ni biologiquement ni autrement non souhaitable et qui est acceptable pour une utilisation dermatologique ou cosmétique, notamment par application topique au niveau de la peau.

Par « application topique », on entend, au sens de la présente invention, une application sur la peau (dont le cuir chevelu), et les muqueuses, de préférence sur la peau, par exemple sur la peau du visage.

Par « extrait sec », on entend, au sens de la présente invention, un extrait dépourvu de solvant d'extraction ou en contenant uniquement à l'état de trace non significative. Un tel extrait sec contient ainsi uniquement de la matière issue de *Silybum marianum* (L.) Gaertn.. Il peut contenir également des traces non significatives de solvant d'extraction.

Par « solvant organique non miscible à l'huile issue d'akènes de *Silybum marianum* (L.) Gaertn. », on entend, au sens de la présente invention, un solvant organique qui n'est pas capable de se mélanger, ou seulement partiellement, avec l'huile issue d'akènes de *Silybum marianum* (L.) Gaertn., de sorte que le mélange du solvant organique et de l'huile issue d'akènes de *Silybum marianum* (L.) Gaertn. donne un mélange hétérogène dans lequel il peut être observé au moins deux phases distinctes.

Par « alcool en C1 à C3 », on entend, au sens de la présente invention, un alcool de formule R²OH dont la chaîne R² est une chaîne hydrocarbonée saturée, linéaire ou ramifiée, comprenant 1 à 3 atomes de carbone. Il pourra s'agir par exemple du méthanol, de l'éthanol, du n-propanol ou de l'isopropanol, en particulier du méthanol, de l'éthanol ou de l'isopropanol. De préférence, il s'agira de l'isopropanol.

Par « température ambiante », on entend, au sens de la présente invention, une température comprise entre 15 et 40°C, de préférence entre 20 et 30°C, notamment d'environ 25°C.

### Silybum marianum (L.) Gaertn.

Le nom scientifique *Silybum marianum* (L.) Gaertn. désigne une plante appartenant à la famille des Asteraceae, annuelle ou bisannuelle, à tige robuste pouvant atteindre plus d'un mètre de hauteur. Ses grandes feuilles luisantes, alternées, sans stipule sont marbrées de blanc et bordées d'épines dures et pointues. Les fleurs sont groupées en capitules terminaux, souvent solitaires. Ils sont entourés de grandes bractées épineuses à l'extrémité très acérée. Les fleurs, tubulées, à cinq lobes, sont de couleur pourpre violacé. Les fruits sont des akènes luisants, noirs ou marbrés de jaune, surmontés d'une aigrette à soies denticulées en anneau à leur base. Le nom vernaculaire de cette plante est Chardon-Marie.

L'akène (souvent dénommé graine de manière erronée dans la littérature) de *Silybum marianum* (L.) Gaertn. et ses préparations sont traditionnellement utilisées par voie orale, dans le traitement symptomatique des troubles fonctionnels digestifs attribués à une origine hépatique.

Le principe actif principal de l'akène de *Silybum marianum* (L.) Gaertn. est la silymarine, mélange de plusieurs flavonolignanes (principalement silybine, isosilybine, silychristine et silydianine). Les akènes contiennent jusqu'à 3% en poids de silymarine. ils sont également constitués d'huile (20-30% en poids), de mucilages et de protéines.

La silymarine a fait l'objet de nombreuses études (*in vitro, in vivo* et cliniques) ayant démontré ses propriétés antioxydante, hépatoprotectrice, digestive, ou encore anti-inflammatoire. Actuellement, des extraits d'akènes de *Silybum marianum* (L.) Gaertn. titrés en silymarine sont présents dans plusieurs préparations pharmaceutiques destinées au traitement de divers troubles hépatiques et biliaires, telles que le Legalon^{®}. La silymarine a fait par ailleurs l'objet d'une étude dans le traitement de l'acné (Sahib et al. 2012), ainsi que dans le traitement de la rosacée érythémato-télangiectasique en association avec du méthylsulfonylméthane (MSM) (Berardesca et al. 2008).

L'huile de *Silybum marianum* (L.) Gaertn. riche en oméga 6 et en vitamine E, est principalement utilisée en cuisine. Elle est obtenue classiquement à partir des akènes par pression à froid. Des études sur les propriétés antioxydante et hépatoprotectrice d'une telle huile de Chardon-Marie, administrée par voie orale, ont cependant été réalisées *in vivo* sur des rats ou des souris (Hermenean et al. 2015 ; Zhu et al. 2014).

Par ailleurs, un extrait d'akènes de Silybum marianum (L.) Gaertn., comprenant moins de 0,2% en poids de silymarine par rapport au poids de l'extrait sec est déjà décrit dans la demande de brevet FR 3 053 253, cet extrait est efficace dans le traitement de l'acné, de la séborrhée, de la rosacée et/ou de la dermite séborrhéique. Dans cette demande de brevet, les inventeurs se focalisent uniquement sur des lésions inflammatoires. Il est mis également en avant l'activité inhibitrice de cet extrait sur l'expression de gènes d'enzymes impliquées dans la synthèse de lipides spécifiques du sébum. Toutefois, aucun effet sur les rougeurs, les flushs et autres dysfonctions vasculaires n'est reporté dans cette demande de brevet.

### Extrait selon l'invention

L'extrait selon l'invention est un extrait d'akènes de *Silybum marianum* (L.) Gaertn. comprenant moins de 0,2%, de préférence moins de 0,1%, en poids de silymarine par rapport au poids de l'extrait sec.

Selon un mode de réalisation particulier, l'extrait selon l'invention contient au moins 0,5% en poids, de préférence au moins 1,0% en poids de béta-sitostérol par rapport au poids de l'extrait sec. En particulier, l'extrait selon l'invention contient entre 0,5% et 2,5% en poids, notamment entre 1,0% et 2,0% en poids, par exemple environ 1,5% en poids de béta-sitostérol par rapport au poids de l'extrait sec. Le rapport massique silymarine/béta-sitostérol de l'extrait selon l'invention pourra être en particulier inférieur à 0,4, notamment inférieur à 0,07. L'extrait selon l'invention pourra comprendre par ailleurs entre 2% et 7% en poids, notamment entre 3% et 6% en poids, par exemple entre 3% et 5% en poids de stérols par rapport au poids de l'extrait sec.

Selon un mode de réalisation particulier, l'extrait selon l'invention contient au moins 3% en poids, de préférence au moins 4% en poids d'acide linoléique libre par rapport au poids de l'extrait sec. En particulier, l'extrait selon l'invention contient entre 3% et 25% en poids, par exemple entre 3 et 20% en poids, notamment entre 3% et 15% en poids, notamment entre 4% et 10% en poids, en particulier entre 4% et 6% en poids, par exemple environ 5% en poids d'acide linoléique libre par rapport au poids de l'extrait sec. L'extrait selon l'invention pourra comprendre par ailleurs entre 10% et 70% en poids, notamment entre 10% et 50% en poids, en particulier entre 10% et 30% en poids, notamment entre 15% et 25% en poids d'acides gras libres par rapport au poids de l'extrait sec.

Selon un autre mode de réalisation particulier, l'extrait selon l'invention contient entre 0,5% et 2,5% en poids, notamment entre 1,0% et 2,0% en poids, par exemple environ 1,5% en poids de béta-sitostérol par rapport au poids de l'extrait sec et entre 3% et 25% en poids, par exemple entre 3 et 20% en poids, notamment entre 3% et 15% en poids, notamment entre 4% et 10% en poids, en particulier entre 4% et 6% en poids, par exemple environ 5% en poids d'acide linoléique libre par rapport au poids de l'extrait sec. Le rapport massique silymarine/béta-sitostérol de l'extrait selon l'invention pourra être en particulier inférieur à 0,4, notamment inférieur à 0,07. L'extrait selon l'invention pourra comprendre par ailleurs entre 2% et 7% en poids, notamment entre 3% et 6% en poids, par exemple entre 3% et 5% en poids de stérols par rapport au poids de l'extrait sec et entre 10% et 70% en poids, notamment entre 10% et 50% en poids, en particulier entre 10% et 30% en poids, notamment entre 15% et 25% en poids d'acides gras libres par rapport au poids de l'extrait sec.

Selon un mode de réalisation particulier, l'extrait selon l'invention contient au moins 0,01% en poids, notamment au moins 0,05% en poids de tocophérols par rapport au poids de l'extrait sec. En particulier, l'extrait selon l'invention contient entre 0,01% et 2% en poids, plus particulièrement entre 0,01% et 1% en poids, encore plus particulièrement entre 0,01% et 0,5% en poids, notamment entre 0,05% et 0,2% en poids, par exemple environ 0,1% en poids de tocophérols par rapport au poids de l'extrait sec. Le rapport massique silymarine/tocophérols de l'extrait selon l'invention pourra être en particulier inférieur à 1, notamment inférieur à 0,1.

Selon un autre mode de réalisation particulier, l'extrait selon l'invention contient entre 0,5% et 2,5% en poids, notamment entre 1,0% et 2,0% en poids, par exemple environ 1,5% en poids de béta-sitostérol par rapport au poids de l'extrait sec ; entre 3% et 25% en poids, par exemple entre 3 et 20% en poids, notamment entre 3% et 15% en poids, notamment entre 4% et 10% en poids, en particulier entre 4% et 6% en poids, par exemple environ 5% en poids d'acide linoléique libre par rapport au poids de l'extrait sec ; et entre 0,01% et 2% en poids, plus particulièrement entre 0,01% et 1% en poids, encore plus particulièrement entre 0,01% et 0,5% en poids, notamment entre 0,05% et 0,2% en poids, par exemple environ 0,1% en poids de tocophérols par rapport au poids de l'extrait sec. L'extrait selon l'invention pourra comprendre par ailleurs entre 2% et 7% en poids, notamment entre 3% et 6% en poids, par exemple entre 3% et 5% en poids de stérols par rapport au poids de l'extrait sec et entre 10% et 70% en poids, notamment entre 10% et 50% en poids, en particulier entre 10% et 30% en poids, notamment entre 15% et 25% en poids d'acides gras libres par rapport au poids de l'extrait sec. Le rapport massique silymarine/béta-sitostérols de l'extrait selon l'invention pourra être en particulier inférieur à 0,4, notamment inférieur à 0,07. Le rapport massique silymarine/tocophérols de l'extrait selon l'invention pourra être en particulier inférieur à 1, notamment inférieur à 0,1.

De préférence, l'extrait selon la présente invention sera un extrait sec ou un extrait, de préférence sec, en mélange avec un solvant autre que le solvant d'extraction, de préférence avec l'octyldodécanol.

Selon un mode de réalisation préféré, l'extrait selon l'invention est obtenu ou est susceptible d'être obtenu par un procédé selon l'invention décrit ci-après.

### Procédé de préparation de l'extrait selon l'invention

Un procédé de préparation d'un extrait selon l'invention comprend une étape d'extraction d'une huile issue d'akènes de *Silybum marianum* (L.) Gaertn. par un solvant d'extraction comprenant, notamment constitué par, une solution aqueuse hydrotropique, de l'eau subcritique ou un solvant organique non miscible à l'huile issue d'akènes de *Silybum marianum* (L.) Gaertn. éventuellement en mélange avec de l'eau.

Selon un mode de réalisation particulier, le solvant d'extraction comprend, notamment est constitué par, un solvant organique non miscible à l'huile issue d'akènes de *Silybum marianum* (L.) Gaertn. éventuellement en mélange avec de l'eau.

Le solvant organique non miscible à l'huile issue d'akènes de *Silybum marianum* (L.) Gaertn. pourra être notamment un alcool en C1 à C3.

Le solvant d'extraction pourra être notamment un alcool en C1 à C3 (tel que le méthanol, l'éthanol ou l'isopropanol) éventuellement en mélange avec de l'eau.

Le solvant organique non miscible à l'huile issue d'akènes de *Silybum marianum* (L.) Gaertn., en particulier un alcool en C1 à C3 tel que le méthanol, l'éthanol ou l'isopropanol, pourra être utilisé en mélange avec de l'eau, notamment dans un rapport volumique solvant organique/eau compris entre 80/20 et 100/0, notamment compris entre 85/15 et 95/5, en particulier d'environ 90/10.

Le solvant d'extraction pourra notamment être choisi parmi le méthanol, un mélange méthanol/eau, l'éthanol, un mélange éthanol/eau, l'isopropanol et un mélange isopropanol/eau. Selon un mode de réalisation préféré, le solvant d'extraction sera du méthanol, un mélange éthanol/eau dans un rapport volumique d'environ 90/10 ou un mélange isopropanol/eau dans un rapport volumique d'environ 90/10, de préférence un mélange isopropanol/eau dans un rapport volumique d'environ 90/10.

L'étape d'extraction de l'huile issue d'akènes de *Silybum marianum* (L.) Gaertn. sera effectuée en particulier par mélange de l'huile issue d'akènes de *Silybum marianum* (L.) Gaertn. avec le solvant d'extraction durant 1 h à 12 h et en particulier à une température comprise entre 15°C et 25°C, notamment d'environ 20°C. La quantité de solvant d'extraction utilisé pour effectuer cette extraction sera avantageusement de 0,5 g à 3 g, en particulier de 1 g à 3 g pour 1 g d'huile issue d'akènes de *Silybum marianum* (L.) Gaertn..

Une phase d'extraction et une phase lipidique seront alors obtenues à la fin de cette extraction. La phase d'extraction sera séparée de la phase lipidique et récupérée avant d'être totalement ou partiellement séchée, notamment sous vide et de préférence en présence d'octyldodécanol. De préférence, la phase d'extraction est totalement séchée, de préférence en présence d'octyldodécanol, notamment sous vide, pour éliminer totalement le solvant d'extraction et obtenir un extrait sec en mélange avec de l'octydodécanol.

L'huile issue d'akènes de *Silybum marianum* (L.) Gaertn. pourra être avantageusement obtenue par extraction à partir d'akènes de *Silybum marianum* (L.) Gaertn. (les akènes pourront être entiers ou en morceaux), notamment par pression, avantageusement par pression à froid, c'est-à-dire sans chauffage, à température ambiante.

Selon un mode de réalisation selon l'invention, le procédé selon l'invention comprendra les deux étapes successives suivantes :
(i) extraction d'une huile à partir d'akènes de *Silybum marianum* (L.) Gaertn., et
(ii) extraction de l'huile issue d'akènes de *Silybum marianum* (L.) Gaertn. avec un solvant d'extraction, comprenant, notamment constitué par, une solution aqueuse hydrotropique, de l'eau subcritique ou un solvant organique non miscible à l'huile issue d'akènes de *Silybum marianum* (L.) Gaertn. éventuellement en mélange avec de l'eau.

Selon un mode de réalisation préféré selon l'invention, le procédé selon l'invention comprendra les étapes successives suivantes :
(i) éventuellement extraction d'une huile à partir d'akènes de *Silybum marianum* (L.) Gaertn.,
(ii) extraction de l'huile issue d'akènes de *Silybum marianum* (L.) Gaertn. avec un solvant d'extraction, comprenant, notamment constitué par, une solution aqueuse hydrotropique, de l'eau subcritique ou un solvant organique non miscible à l'huile issue d'akènes de *Silybum marianum* (L.) Gaertn. éventuellement en mélange avec de l'eau,
(iii) récupération de la phase d'extraction obtenue à l'étape (ii), et
(iv) séchage partiel ou total de la phase d'extraction pour donner un extrait concentré ou sec selon l'invention, de préférence séchage total de la phase d'extraction en présence d'octyldodécanol pour donner un extrait sec selon l'invention en mélange avec de l'octyldodécanol.

L'étape (i) sera avantageusement réalisée par pression à froid des akènes de *Silybum marianum* (L.) Gaertn., entiers ou en morceaux.

L'étape (ii) sera avantageusement réalisée avec un solvant d'extraction tel que défini précédemment, et notamment choisi parmi le méthanol, un mélange méthanol/eau, l'éthanol, un mélange éthanol/eau, l'isopropanol, et un mélange isopropanol/eau.

Le solvant organique non miscible à l'huile issue d'akènes de *Silybum marianum* (L.) Gaertn., en particulier un alcool en C1 à C3 tel que le méthanol, l'éthanol ou l'isopropanol, pourra être utilisé en mélange avec de l'eau, notamment dans un rapport volumique solvant organique/eau compris entre 80/20 et 100/0, notamment compris entre 85/15 et 95/5, en particulier d'environ 90/10. Un solvant d'extraction avantageux est un mélange isopropanol/eau dans un rapport volumique d'environ 90/10.

L'étape d'extraction (ii) pourra être effectuée par mélange de l'huile issue d'akènes de *Silybum marianum* (L.) Gaertn. avec le solvant d'extraction durant 1 h à 12 h et en particulier à une température de 15°C à 25°C, notamment d'environ 20°C. La quantité de solvant d'extraction utilisé pour effectuer cette extraction sera avantageusement de 0,5 g à 3 g, en particulier de 1 g à 3 g pour 1 g d'huile issue d'akènes de *Silybum marianum* (L.) Gaertn.. Cette étape (ii) d'extraction permet d'obtenir à la fin une phase d'extraction d'intérêt et une phase lipidique. L'étape (iii) sera effectuée avantageusement par séparation de la phase d'extraction de la phase lipidique.

L'étape (iv) sera avantageusement réalisée sous vide, de préférence en présence d' octyldodécanol.

### Composition dermatologique ou cosmétique selon l'invention

La composition dermatologique ou cosmétique selon l'invention comprend un extrait selon l'invention tel que défini précédemment en mélange avec au moins un excipient dermatologiquement ou cosmétiquement acceptable. Il s'agit de préférence d'une composition topique, c'est-à-dire sous une forme apte à une application topique, en particulier sur la peau. Les compositions selon l'invention sont avantageusement destinées à une application topique, et sont donc sous une forme apte à une application topique, en particulier sur la peau, notamment sur le visage.

Les compositions selon l'invention pourront ainsi se présenter sous les formes qui sont habituellement connues pour une administration topique, c'est-à-dire notamment les lotions, les mousses, les gels, les dispersions, les émulsions, les sprays, les sérums, les masques ou les crèmes, avec des excipients permettant notamment une pénétration cutanée afin d'améliorer les propriétés et l'accessibilité du principe actif. Avantageusement, il s'agira d'une crème. Ces compositions contiennent généralement, outre l'extrait selon la présente invention, un milieu physiologiquement acceptable, en général à base d'eau ou de solvant, par exemple des alcools, des éthers ou des glycols. Elles peuvent également contenir des agents tensioactifs, des agents complexants, des conservateurs, des agents stabilisants, des émulsifiants, des épaississants, des gélifiants, des humectants, des émollients, des agents hydratants, des huiles essentielles, des parfums, des oligo-éléments, des colorants, des agents matifiants, des filtres chimiques ou minéraux, des eaux thermales, etc.

La composition comprendra avantageusement de l'octyldodécanol, notamment en une quantité de 10 à 15% en poids par rapport au poids total de la composition. L'octydodécanol est notamment utilisé comme support de l'extrait.

### Applications

L'extrait selon l'invention ou la composition dermatologique ou cosmétique selon l'invention permet en particulier de réguler la voie de signalisation du récepteur tyrosine kinase Tie2/angiopoiétine. L'extrait selon l'invention permet ainsi plus particulièrement de réduire les dysfonctionnements vasculaires.

L'extrait selon l'invention (ou la composition dermatologique ou cosmétique selon l'invention contenant un tel extrait) par cette régulation de la voie de signalisation du récepteur tyrosine kinase Tie2/angiopoiétine permet d'aller vers un état de quiescence vasculaire, c'est-à-dire un état de stabilité vasculaire ou d'équilibre.

L'extrait selon l'invention ou la composition dermatologique ou cosmétique selon l'invention est ainsi utile dans le traitement ou la prévention de rougeurs cutanées.

Les rougeurs cutanées peuvent être liées au stress de l'environnement ou encore associées à un désordre cutané tel que la rosacée, et en particulier la rosacée érythémato-télangiectasique, encore appelée rosacée vasculaire. La rosacée érythémato-télangiectasique est le seul type de rosacée purement vasculaire. Cette rosacée ne présente pas de papules et de pustules.

Les rougeurs cutanées peuvent être liées également à un désordre cutané tel que la dermatite atopique.

L'extrait selon l'invention ou la composition dermatologique ou cosmétique selon l'invention peut donc en particulier être utile chez des patients atteints de rosacée, notamment de rosacée érythémato-télangiectasique, encore appelée rosacée vasculaire.

L'extrait selon l'invention ou la composition dermatologique ou cosmétique selon l'invention peut également être utile chez des patients atteints de dermatite atopique.

Les rougeurs cutanées peuvent être présentes sous forme de flushs. L'extrait selon l'invention ou la composition dermatologique ou cosmétique selon l'invention permet notamment de diminuer la sévérité et/ou la fréquence des flushs. La sévérité des flushs peut être estimée à l'aide de l'échelle GFSS (Global Flushing Severity Score), l'échelle allant de 0 (aucun) à 10 (extrême). La réduction de la fréquence des flushs peut être déterminée à l'aide de l'indice PGA (Patient's Global Assesment), l'indice allant de 1 (aggravation) à 4 (très bonne amélioration).

### FIGURE

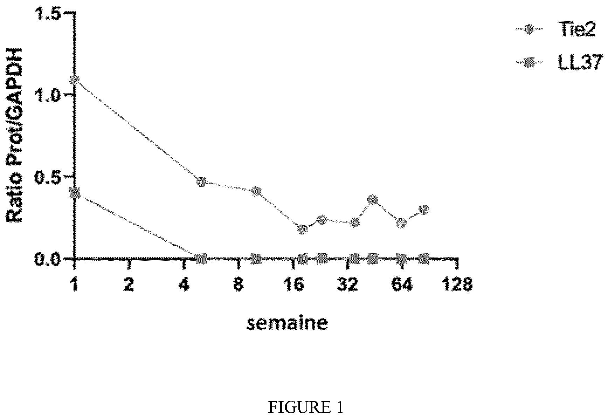

Figure 1 : effets *in vivo* d'un extrait de *Silybum* marianum (L.) Gaertn. selon l'invention sur l'expression des protéines Tie2 et cathélicidine (LL37) chez un patient (exprimée en ratio de protéine / glycéraldéhyde-3-phosphate déshydrogénase (GPADH)).

### EXEMPLES

### Exemple 1 : Préparation d'un extrait de Silybum marianum (L.) Gaertn. selon l'invention

Un extrait selon l'invention en mélange avec de l'octyldodécanol a été préparé selon le procédé suivant :
- pression à froid d'akènes de *Silybum marianum* (L.) Gaertn. pour obtenir une huile issue d'akènes de *Silybum marianum* (L.) Gaertn.,
- extraction de l'huile issue d'akènes de *Silybum marianum* (L.) Gaertn. par un mélange isopropanol/eau (90/10 v/v) avec 1 gramme du mélange isopropanol / eau par gramme d'huile pendant 2 heures à 20°C,
- récupération de la phase isopropanolique, et
- évaporation du solvant en présence d'octyldodécanol en une quantité suffisante pour obtenir le ratio extrait/octyldodécanol de la Formulation A, éventuellement après standardisation.

### Exemple 2 : Effets in vitro d'un extrait de Silybum marianum (L.) Gaertn. sur l'expression de gènes impliqués dans la quiescence vasculaire

Le but de cette étude est d'évaluer les effets d'un extrait de *Silybum marianum* (L.) Gaertn. selon l'exemple 1 sur l'expression de gènes impliqués dans la quiescence vasculaire, cette dernière étant bien décrite dans l'étude de Ricard et al. 2021.

Les études ont été conduites sur des cultures de plusieurs lignées cellulaires : cellules endothéliales, cellules ombilicales humaines, cellules de papille dermique, fibroblastes dermiques humains, cellules lymphatiques, lignée cellulaire obtenue à partir de glandes sébacées humaines, kératinocytes humains.

Les cellules sont traitées pendant 3 jours consécutifs par l'extrait de *Silybum marianum* (L.) Gaertn. à 300 µg/ml dans une solution aqueuse d'isopropanol à 1% ou par son solvant (solution aqueuse d'isopropanol à 1%).

Au quatrième jour, l'extraction d'ARN est réalisée, l'analyse transcriptomique est faite par RNA-Seq et PCR.

Les résultats, présentés dans le tableau 1 ci-dessous, montrent que, d'une façon très surprenante, une expression différentielle d'un groupe de gènes spécifiquement interactifs dans la voie de signalisation angiopoiétine / Tie2 est induite par l'extrait de *Silybum marianum* (L.) Gaertn., indiquant un ciblage inédit de la fonction de maintien de la quiescence endothéliale.

**[Tableau 1]**

| Gènes | | Facteur de changement* | Valeur p |
|---|---|---|---|
| Sur-exprimés | ANGPTL4 | 5,84 | P<0,0001 |
| | QRFPR | 5,15 | P<0,0001 |
| | ANGPT2 | 4,63 | P<0,0001 |
| | ESM1 | 3,60 | P=0,001 |
| | REM1 | 3,32 | P<0,0001 |
| | UNC13B | 3,14 | P<0,0001 |
| | PCDH20 | 2,76 | P=0,001 |
| | MARCH4 | 2,50 | P<0,0001 |
| | TEK/Tie2 | 2,10 | P=0,003 |
| Sous-exprimés | FGA | -8,65 | P<0,0001 |
| | FGB | -5,55 | P<0,0001 |
| | FGG | -5,54 | P<0,0001 |
| | FGL1 | -3,43 | P<0,0001 |
| | EDN2 | -2,96 | P<0,0001 |
| | KEL | -2,57 | P<0,0001 |
| | RIC3 | -3,26 | P<0,0001 |
| | CNR1 | -3,87 | P<0,0001 |
| | SEMA6D | -3,12 | P<0,0001 |
| | ANGPT4 | -1,80 | P=0,015 |
| | ANGPT1 | -1,16 | P=0,006 |
| | ANKRD1 | -1,34 | P<0,0001 |
| | NEDD9 | -1,06 | P<0,0001 |

| | | | |
|---|---|---|---|
| * Fold change en anglais | | | |

L'extrait de *Silybum marianum* (L.) Gaertn. apparait ainsi être particulièrement adapté pour l'homéostasie vasculaire.

### Exemple 3 : Effets in vivo d'un extrait de Silybum marianum (L.) Gaertn. sur l'expression des protéines Tie2 et LL37

Chez des patients atteints de rosacée traités par l'extrait de *Silybum marianum* (L.) Gaertn. selon l'exemple 1, des prélèvements cutanés sont pratiqués sur le visage avant le début du traitement puis de façon séquentielle pendant plusieurs semaines alors que les patients appliquent cet extrait de *Silybum marianum* (L.) Gaertn. deux fois par jour (matin et soir) à l'aide de la Formulation A avec une concentration en extrait de 1,75% ou 7% selon les cas. Les prélèvements sont faits toujours au même endroit par la méthode de biopsie superficielle au cyanoacrylate. Les protéines sont extraites de la biopsie et le contenu en Tie2, et LL37 est analysé par la méthode de western blot.

Les résultats sont présentés sur la Figure 1 pour un patient.

Cette figure illustre le fait que, chez ce patient traité par l'extrait de *Silybum marianum* (L.) Gaertn. pendant 128 semaines, l'expression du marqueur d'activité de la rosacée, LL37 encore appelé cathélicidine, est réduit de façon durable. Cette protéine (cathélicidine) est en effet surexprimée pour des maladies inflammatoires, comme la rosacée. Il est intéressant de noter que le niveau d'expression du récepteur Tie2 suit la même tendance.

Ces observations permettent (pour la première fois) de faire un lien entre le système Tie2-angiopoiétines et les manifestations vasculaires de la rosacée et sont compatibles avec un effet agoniste de l'extrait selon l'invention.

### Exemple 4 : Exemple de composition selon l'invention

**[Tableau 2]**

| Composants de la Formulation A | Quantité (% en poids) |
|---|---|
| Eau | Qsp |
| Octyldodécanol | 10-15 |
| Alcool myristique | 2-6 |
| Myristyl glucoside | 1-5 |
| Glycérine | 3-10 |
| Extrait d'akènes de *Silybum marianum* (L.) Gaertn. | 1,75 |
| Gomme de xanthane | 0,1-1 |
| Acide citrique | <1 |
| Benzoate de sodium | <1 |

### Exemple 5 : Etude clinique de court terme

### Méthode

Une étude clinique de 4 semaines englobant 44 sujets (de 33 à 65 ans), tous atteints de rosacée érythémato-télangiectasique (stade 2), a été réalisée. Les sujets ont appliqué deux fois par jour la Formulation A (exemple 4) pendant 4 semaines sur les zones les plus touchées, environ une noisette de Formulation A est appliquée par hémi visage.

Trois visites chez le dermatologue sont organisées, à J1, J15 et en fin d'étude à J29. Plusieurs critères sont évalués :
- La sévérité de l'érythème, estimée à l'aide de l'échelle CEA (Clinical Erythema Assessment), l'échelle allant de 1 (aggravation) à 4 (très bonne amélioration) ;
- La sévérité des flushs, estimée à l'aide de l'échelle GFSS (Global Flushing Severity Score), l'échelle allant de 0 (aucun) à 10 (extrême) ;
- La fréquence des flushs, l'amélioration ou l'aggravation étant estimée à l'aide de l'indice PGA (Patient's Global Assessment), l'indice allant de 1 (aggravation) à 4 (très bonne amélioration) ;
- Des signes fonctionnels sont auto-évalués comme l'inconfort, la chaleur, les tiraillements, les picotements, ou les sensations de brûlure.

### Résultats

Le premier point à noter est que la composition A (composition selon l'invention) a été testée pendant 1 mois sous contrôle dermatologique et ophtalmologique sur des peaux à tendance couperosique. Une bonne tolérance cutanée et une excellente tolérance oculaire sont répertoriées par les sujets ayant appliqué cette composition deux fois par jours pendant 1 mois.

Les principaux résultats, moyennés sur l'ensemble des patients, obtenus sur les critères détaillés ci-dessus, sont résumés dans le tableau 3 suivant.

**[Tableau 3]**

| Critères | J1 | J15 | Var J15 vs J1 | J29 | Var J29 vs J1 |
|---|---|---|---|---|---|
| Sévérité de l'érythème | 2,4 | 2,3 | -2,5% SNS | 2,2 | -8,2% * |
| Fréquence des flushs | 2,6 | 2,1 | -18,5% ** | 1,9 | -26,2% ** |
| Sévérité des flushs | 5,0 | 3,5 | -30,6% ** | 2,6 | -48,4% ** |

| | | | | | |
|---|---|---|---|---|---|
| Var : variation ; * p< 0,05 versus J1 ; ** p< 0,001 versus J1 ; SNS : Statistiquement non significatif | | | | | |

Les inventeurs mettent clairement en évidence une efficacité remarquable de la composition selon l'invention sur les critères vasculaires de la rosacée. En effet, les patients atteints de rosacée érythémato-télangiectasique voient que, dès 15 jours de traitement, la fréquence des flushs et leur sévérité diminuent significativement. Après un mois de traitement, une réduction de plus de 25% de la fréquence des flushs est observée et une réduction de leur sévérité atteignant presque 50% est rapportée. La composition selon l'invention apparait donc particulièrement bien adaptée au versant vasculaire de la rosacée érythémato-télangiectasique.

Les résultats observés pour les signes fonctionnels sont résumés dans le tableau 4 ci-dessous.

**[Tableau 4]**

| Critères | J1 | J1imm | Var J1imm vs J1 | J15 | Var J15 vs J1 | J29 | Var J29 vs J1 |
|---|---|---|---|---|---|---|---|
| Inconfort | 6,9 | 6,3 | -9% * | 6,3 | -8% ** | 5,7 | -17% ** |
| Chaleur | 6,5 | 2,1 | -67% ** | 4,2 | -35% ** | 3,2 | -51% ** |
| Tiraillements | 3,0 | 1,0 | -66% ** | 1,8 | -38% ** | 1,2 | -58% ** |
| Picotements | 1,5 | 0,0 | -100% ** | 0,5 | -67% ** | 0,4 | -71% ** |
| Brûlure | 0,9 | 0,0 | -100% ** | 0,6 | -37% SNS | 0,4 | -56% * |

| | | | | | | | |
|---|---|---|---|---|---|---|---|
| Imm : effet immédiat ; Var : variation ; * p< 0,05 versus J1 ; ** p< 0,001 versus J1 ; SNS : Statistiquement non significatif | | | | | | | |

Les signes fonctionnels sont également bien améliorés avec 1 mois de traitement par l'application deux fois par jours de la composition A selon l'invention. Les effets immédiats sont très importants, mais l'amélioration des ces signes fonctionnels dure également dans le temps. Cette composition selon l'invention apparait particulièrement bien adaptée pour de longues périodes de traitement.

### Exemple 6 : Etude clinique de long-terme

Une étude clinique a été réalisée sur 32 patients souffrant de rosacée érythémato-télangiectasique pour étudier l'effet d'une application topique d'une composition selon l'invention, sur un indice global de l'état clinique et la rougeur. Ces patients ont appliqué cette composition sur une durée relativement longue, d'au moins 8 semaines de traitement à 64 semaines.

Cette étude a pour but de vérifier l'efficacité à long terme de cette composition selon l'invention.

L'analyse des effets thérapeutiques obtenus a été effectuée par un dermatologue expert clinique selon la méthode d'analyse globale de l'état clinique IGA (Investigator Global Assessment) acceptée par la FDA (Food and Drug Administration) et adaptée à la rosacée.

L'indice de rougeur est estimé par le dermatologue expert par un système de score tenant compte de la présence ou non et de la sévérité des érythèmes transitoires (flushs), des érythèmes non transitoires, des oedèmes, des changements phymateux et de telangiectasia.

Les résultats sur l'IGA sont détaillés dans le tableau 5 ci-dessous. Les scores d'IGA sont calculés en % de la valeur déterminée la semaine 1.

**[Tableau 5]**

| Patients | Semaines de traitement | | | | | | |
|---|---|---|---|---|---|---|---|
| | 1 | 2 | 4 | 8 | 16 | 32 | 64 |
| 1 | 100 | 100 | 100 | 100 | - | - | - |
| 2 | 100 | 100 | 100 | 66,7 | - | - | - |
| 3 | 100 | 100 | - | - | - | - | - |
| 4 | 100 | 66,7 | 66,7 | 33,3 | - | - | - |
| 5 | 100 | 100 | 100 | 92,6 | 58,4 | 0 | 24,4 |
| 6 | 100 | 100 | 100 | 100 | 66,7 | - | - |
| 7 | 100 | 100 | 100 | 100 | 100 | 33,3 | - |
| 8 | 100 | 100 | 100 | 100 | 75 | 75 | 50 |
| 9 | 100 | 100 | 100 | 100 | 100 | 100 | 100 |
| 10 | 100 | 83,3 | 50 | 50 | 85,7 | 0 | - |
| 11 | 100 | 100 | 100 | 25 | 0 | 0 | - |
| 12 | 100 | 100 | 100 | - | - | - | - |
| 13 | 100 | 100 | 100 | 50 | - | - | - |
| 14 | 100 | 85 | 40 | 47,5 | 40,4 | 25 | 35 |
| 15 | 100 | 100 | 100 | - | - | - | - |
| 16 | 100 | 75 | 50 | 50 | 50 | - | - |
| 17 | 100 | 100 | 100 | 100 | 100 | 100 | - |
| 18 | 100 | 100 | 100 | 100 | - | - | - |
| 19 | 100 | 100 | 83,3 | 66,7 | 66,7 | 39,6 | 64,2 |
| 20 | 100 | 100 | 100 | 73,4 | 66,7 | 33,3 | - |
| 21 | 100 | 100 | 100 | 66,7 | 14,3 | 32,3 | 26,1 |
| 20 | 100 | 50 | 50 | 0 | - | - | - |
| 22 | 100 | 100 | 66,7 | - | - | - | - |
| 23 | 100 | 100 | 100 | 66,7 | 16,7 | 33,3 | - |
| 24 | 100 | 66,7 | 33,3 | - | - | - | - |
| 25 | 100 | 94,4 | 83,3 | - | - | - | - |
| 26 | 100 | 100 | 93,3 | 66,7 | 33,3 | 0 | - |
| 27 | 100 | 100 | 100 | 98,2 | 90,9 | 78,2 | - |
| 28 | 100 | 100 | 100 | 66,7 | - | - | - |
| 29 | 100 | 100 | 100 | 73,4 | 66,7 | 33,3 | - |
| 30 | 100 | 100 | 100 | 100 | 100 | 100 | - |
| 31 | 100 | 96,9 | 90,6 | 78,1 | 75 | 75 | - |
| 32 | 100 | 100 | 100 | 86,4 | 50 | 50 | - |
| nombre de patients | 32 | 32 | 31 | 26 | 19 | 17 | 6 |
| Moyenne (%) | 100 | 94,3 | 87,3 | 72,5 | 62,6 | 45,6 | 48,3 |
| esm | 0,0 | 2,2 | 3,8 | 5,4 | 7,1 | 8,8 | 12,3 |

| | | | | | | | |
|---|---|---|---|---|---|---|---|
| Esm : erreur standard à la moyenne | | | | | | | |

Les résultats sur l'indice de rougeur sont détaillés dans le tableau 6 ci-dessous. Les scores de rougeur sont calculés en % de la valeur déterminée la semaine 1

**[Tableau 6]**

| Patients | Semaines de traitement | | | | | | |
|---|---|---|---|---|---|---|---|
| | 1 | 2 | 4 | 8 | 16 | 32 | 64 |
| 1 | 100 | 100 | 100 | 92,3 | - | - | - |
| 2 | 100 | 62,5 | 62,5 | 50 | - | - | - |
| 3 | 100 | 75 | - | - | - | - | - |
| 4 | 100 | 33,3 | 33,3 | 0 | - | - | - |
| 5 | 100 | 95,3 | 86 | 68,7 | 47,7 | 18,2 | 15,8 |
| 6 | 100 | 81,3 | 56,3 | 50 | 37,5 | - | - |
| 7 | 100 | 100 | 100 | 77,3 | 60,2 | 27,3 | - |
| 8 | 100 | 88,3 | 72,6 | 68,2 | 47,1 | 39,4 | 17,6 |
| 9 | 100 | 100 | 100 | 100 | 100 | 100 | 100 |
| 10 | 100 | 83,3 | 50 | 50 | 45,2 | 16,7 | - |
| 11 | 100 | 100 | 100 | 62,5 | 50 | 50 | - |
| 12 | 100 | 77,8 | 66,7 | - | - | - | - |
| 13 | 100 | 66,7 | 50 | 50 | - | - | - |
| 14 | 100 | 79,2 | 37,5 | 31,6 | 25 | 19,5 | 20,2 |
| 15 | 100 | 85,7 | 71,4 | - | - | - | - |
| 16 | 100 | 72,4 | 44,4 | 44,4 | 34,9 | - | - |
| 17 | 100 | 100 | 100 | 100 | 88,3 | 61,7 | - |
| 18 | 100 | 95 | 81,7 | 70 | - | - | - |
| 19 | 100 | 100 | 80 | 40 | 40 | 40 | 40 |
| 20 | 100 | 94,4 | 83,3 | 56,7 | 50 | 33,3 | - |
| 21 | 100 | 93,3 | 80 | 40 | 8,6 | 0 | 0 |
| 20 | 100 | 44,4 | 22,2 | 0 | - | - | - |
| 22 | 100 | 70 | 50 | - | - | - | - |
| 23 | 100 | 87,9 | 63,6 | 36,4 | 9,1 | 0 | - |
| 24 | 100 | 72,7 | 54,5 | - | - | - | - |
| 25 | 100 | 95 | 85 | - | - | - | - |
| 26 | 100 | 91,7 | 77,3 | 58,3 | 33,3 | 16,7 | - |
| 27 | 100 | 106,7 | 118 | 130,6 | 120,9 | 103,9 | - |
| 28 | 100 | 92,6 | 77,8 | 44,4 | - | - | - |
| 29 | 100 | 100 | 100 | 100 | 105,4 | 130,7 | - |
| 30 | 100 | 93,4 | 80,2 | 53,7 | 32,4 | 31,4 | - |
| 31 | 100 | 87,5 | 75 | 66,7 | 25 | 12,5 | - |
| 32 | 100 | 93,3 | 80 | 40 | 8,6 | 0 | 0 |
| nombre de patients | 32 | 32 | 31 | 26 | 19 | 17 | 6 |
| Moyenne (%) | 100,0 | 85,2 | 72,9 | 59,3 | 50,6 | 41,3 | 32,3 |
| esm | 0,0 | 2,9 | 4,1 | 5,8 | 7,2 | 9,1 | 14,5 |

| | | | | | | | |
|---|---|---|---|---|---|---|---|
| Esm : erreur standard à la moyenne | | | | | | | |

Les inventeurs ont mis en évidence à l'aide de cette étude, qu'une composition selon l'invention est très efficace chez des patients atteints de rosacée érythémato-télangiectasique. En effet, l'IGA baisse dès 4 semaines de traitements, et cette réduction est régulière en fonction de la durée du traitement. Au niveau des rougeurs, l'effet bénéfique de la composition selon l'invention est encore plus marqué. En effet, après 8 semaines de traitement, on peut observer environ 30% de réduction de l'IGA et près de 50% de réduction du score des rougeurs. La cible prioritaire de cette composition semble bien être vasculaire, comme le laissait entendre les résultats de l'exemple 2, sur les gènes impliqués dans l'angiogénèse.

Il apparait donc que cette composition selon l'invention est tout particulièrement bénéfique chez les patients atteints de rosacée érythémato-télangiectasique en réduisant les dysfonctionnements vasculaires de cette pathologie.

Les inventeurs mettent ainsi en évidence que cette composition est capable de réguler, puis de maintenir sur du long terme, la quiescence du réseau vasculaire cutané des patients atteints de rosacée érythémato-télangiectasique.

Par ailleurs, chez ces patients, lorsque les rougeurs étaient fortement réduites après plusieurs semaines de traitement, il est à noter qu'aucune récidive n'a été observée.

La composition selon l'invention apparait ainsi également limiter la réapparition de ces rougeurs.

### Références bibliographiques

· FR 3 053 253.
· Akwii et al. 2019, Cells, 8, 471.
· Berardesca et al. 2008, Journal of Cosmetic Dermatology, 7, 8-14.
· Edwards 1980, Br. J. Vener. Dis., 56, 285-290.
· Hermenean et al. 2015, Open Life Sci., 10(1), 225-236.
· Kuki et al. 2012, Chromatographia, 75, 175-180.
· Lee et al. 2021, Int. J. Mol. Sci., 221, 12035.
· Ricard et al. 2021, Nature Reviews Cardiology, 18, 565-580.
· Saharinen et al. 2017, Nature Reviews Drug Discovery, 16, 635-661.
· Sahib et al. 2012, J. Clin. Exp. Dermatol. Res., 3, 5.
· Zhu et al. 2014, Pharmacogn Mag., 10 (Suppl. 1), S92-S99.

## Revendications

1. Extrait d'akènes de *Silybum marianum* (L.) Gaertn. comprenant moins de 0,2%, de préférence moins de 0,1%, en poids de silymarine par rapport au poids de l'extrait sec, et susceptible d'être obtenu par un procédé comprenant une étape d'extraction d'une huile issue d'akènes de *Silybum marianum* (L.) Gaertn. avec un solvant d'extraction comprenant une solution aqueuse hydrotropique, de l'eau subcritique ou un solvant organique non miscible à l'huile issue d'akènes de *Silybum marianum* (L.) Gaertn. éventuellement en mélange avec de l'eau, pour son utilisation dans le traitement ou la prévention de rougeurs cutanées chez des patients atteints de rosacée érythémato-télangiectasique et/ou de dermatite atopique.

2. Extrait pour utilisation selon la revendication 1, **caractérisé en ce que** l'extrait est un extrait sec, éventuellement en mélange avec de l'octyldodécanol.

3. Extrait pour utilisation selon l'une quelconque des revendications 1 et 2, **caractérisé en ce que** le solvant d'extraction est un alcool en C1 à C3, de préférence choisi parmi le méthanol, l'éthanol et l'isopropanol, éventuellement en mélange avec de l'eau.

4. Extrait pour utilisation selon l'une quelconque des revendications 1 à 3, **caractérisé en ce que** les rougeurs cutanées sont présentes chez des patients atteints de rosacée érythémato-télangiectasique.

5. Extrait pour utilisation selon l'une quelconque des revendications 1 à 3, **caractérisé en ce que** les rougeurs cutanées sont présentes chez des patients atteints de dermatite atopique.

6. Extrait pour utilisation selon l'une quelconque des revendications 1 à 5, **caractérisé en ce que** les rougeurs cutanées sont présentes sous forme de flushs.

7. Extrait pour utilisation selon la revendication 6, **caractérisé en ce que** le traitement ou la prévention des rougeurs cutanées permet de diminuer la sévérité des flushs.

8. Extrait pour utilisation selon la revendication 6 ou 7, **caractérisée en ce que** le traitement ou la prévention des rougeurs cutanées permet de diminuer la fréquence des flushs.

9. Extrait pour utilisation selon l'une quelconque des revendications 1 à 8, **caractérisé en ce que** la voie de signalisation du récepteur tyrosine kinase Tie2/angiopoiétine est régulée.

10. Composition dermatologique ou cosmétique comprenant un extrait tel que défini à l'une quelconque des revendications 1 à 3 en mélange avec au moins un excipient dermatologiquement ou cosmétiquement acceptable pour son utilisation dans le traitement ou la prévention de rougeurs cutanées chez des patients atteints de rosacée érythémato-télangiectasique et/ou de dermatite atopique.

11. Composition dermatologique ou cosmétique pour utilisation selon la revendication 10, **caractérisée en ce que** la composition est sous une forme apte à une application topique, en particulier sur la peau.

12. Composition dermatologique ou cosmétique pour utilisation selon l'une quelconque des revendications 10 et 11, **caractérisée en ce que** les rougeurs cutanées sont présentes chez des patients atteints de rosacée érythémato-télangiectasique.

13. Composition dermatologique ou cosmétique pour utilisation selon l'une quelconque des revendications 10 et 11, **caractérisée en ce que** les rougeurs cutanées sont présentes chez des patients atteints de dermatite atopique.

14. Composition dermatologique ou cosmétique pour utilisation selon l'une quelconque des revendications 10 à 13, **caractérisée en ce que** les rougeurs cutanées sont présentes sous forme de flushs.

15. Composition dermatologique ou cosmétique pour utilisation selon la revendication 14, **caractérisée en ce que** le traitement ou la prévention des rougeurs cutanées permet de diminuer la sévérité des flushs.

16. Composition dermatologique ou cosmétique pour son utilisation selon la revendication 14 ou 15, **caractérisée en ce que** le traitement ou la prévention des rougeurs cutanées permet de diminuer la fréquence des flushs.

17. Composition dermatologique ou cosmétique pour utilisation selon l'une quelconque des revendications 10 à 16, **caractérisée en ce que** la voie de signalisation du récepteur tyrosine kinase Tie2-angiopoiétine est régulée.
